# EUROPEAN PATENT APPLICATION

(11) **EP 4 708 326 A1**
(43) Date of publication of application: **11.03.2026**
(21) Application number: 25201082.2
(22) Date of filing: 09.09.2025
(51) Int. Cl.: G16H 50/70, G16H 50/20, G16H 10/20, A61B 5/00, G16H 20/10, G16H 20/70, G16H 50/30, A61B 5/16

(54) **DEVICE FOR GENERATING AN ARTIFICIAL INTELLIGENCE SYSTEM FOR MONITORING A PATIENT SUFFERING FROM PSYCHIATRIC DISORDERS THROUGH SPEECH ANALYSIS**

(30) Priority: 09.09.2024 FR 2409558
(71) Applicant: Callyope, 92200 Neuilly-sur-Seine (FR)
(72) Inventor: RIAD, Rachid, 75013 Paris (FR); CAO, Xuan-Nga, 92210 Saint-Cloud (FR); DENAIS, Martin, 75018 Paris (FR); LESAGE, Adrien, 75012 Paris (FR); DE GENNES, Marc, 75010 Paris (FR); ROQUEFORT, Filomène, 75010 Paris (FR); ANDÓ, Angelika, 92340 Bourg-la-Reine (FR)
(74) Representative: Icosa

(57) **Abstract**

The invention relates to a device and a computer-implemented method for generating an artificial intelligence system (31) for monitoring a patient suffering from psychiatric disorders, said device (1) comprising: at least one input configured to receive (11) a training dataset (21), a pretrained biomedical language model (24) (LLM), and an initial architecture (20) of said artificial intelligence system (31) for monitoring, said initial architecture (20) comprising a first medical text encoder and a second encoder of vocal and linguistic signals; at least one processor configured to generate (13) said artificial intelligence system (31) for monitoring by jointly training said first encoder and said second encoder of said initial architecture (20) based on said training dataset (21); at least one output configured to provide as output said artificial intelligence system (31) for monitoring.

## Description

### FIELD OF THE INVENTION

The present invention relates to a method and a device for monitoring a patient suffering from psychiatric disorders using artificial intelligence. More specifically, the invention relates to a method and a device for generating an artificial intelligence system for monitoring a patient suffering from psychiatric disorders through speech analysis.

### BACKGROUND OF THE INVENTION

Vocal biomarkers emerge as a promising avenue for evaluating mental health due to their unique characteristics. These markers have potential as non-invasive, cost-effective, and practical tools. Recent advances have significantly simplified and reduced the cost of acquiring vocal data, making them more accessible compared to traditional biological, imaging, or cognitive markers. Additionally, collecting vocal data requires minimal effort from patients and clinicians and can even be done remotely, further enhancing its feasibility in various contexts.

However, the use of vocal biomarkers faces several significant limitations. Firstly, it remains fragmented, covering only specific aspects of mental health. Secondly, progress is hindered by the insufficient number of well-annotated vocal data from patients, given that collecting these data, along with high-quality psychiatric evaluations, is costly. Finally, the scale of current vocal datasets is far below the standards common in other machine learning and signal processing domains, particularly structured labels.

The present invention aims to address these existing limitations in the field.

### SUMMARY

The invention relates to a device for generating an artificial intelligence system for monitoring a patient suffering from psychiatric disorders, said device comprising:
- at least one input configured to receive:
   ∘ a training dataset, comprising a plurality of training samples for a plurality of subjects, each training sample of said dataset comprising, for each subject of said plurality of subjects:
      ▪ a vocal signal of said subject;
      ▪ clinical information related to said subject comprising at least: a result of a psychiatric questionnaire, at least one prescription of at least one treatment, at least one concentration of a treatment previously measured from a blood sample, and/or demographic data;
   ∘ a pretrained biomedical language model (LLM for Large Language Model) configured to receive as input, for a patient, clinical information related to said patient comprising at least: a result of a psychiatric questionnaire, at least one prescription of at least one treatment, at least one concentration of a treatment previously measured from a blood sample, and/or demographic data; said biomedical language model being further configured to generate as output a message summarizing the clinical situation of said patient;
   ∘ an initial architecture of said artificial intelligence system for monitoring, said initial architecture being configured to provide at least one prediction of symptoms, a dosage of at least one treatment prescribed to a patient in psychiatry, and/or transcriptions, said initial architecture comprising:
      ▪ a first medical text encoder configured to receive as input a message summarizing the clinical situation of said patient generated by said biomedical language model, and generate as output a vector of medical text features;
      ▪ a second encoder of vocal and linguistic signals configured to receive as input at least one vocal signal of a patient and generate as output a vector of vocal and linguistic features, said second encoder being obtained by a pretrained foundation model;

   - at least one processor configured to generate said artificial intelligence system for monitoring by jointly training said first encoder and said second encoder of said initial architecture based on said training dataset, wherein the joint training comprises iterating until convergence, for each training sample, the following steps:
      ∘ obtaining a message summarizing the clinical situation of said patient by said biomedical language model receiving as input at least part of the clinical information comprised in the training sample;
      ∘ obtaining a vector of medical text features by said first medical text encoder receiving as input the message summarizing the clinical situation;
      ∘ obtaining a vector of vocal and linguistic features by said second encoder of vocal and linguistic signals receiving as input the at least one vocal signal comprised in the training sample;
      ∘ modifying the parameters of the initial architecture so as to minimize a loss function based on a multimodality matrix, having at least two dimensions, defined from said vector of medical text features of said first encoder and said vector of vocal and linguistic features of said second encoder;
   - at least one output configured to provide, as output, said artificial intelligence system for monitoring.

In other words the step of modifying the parameters of the initial architecture implies that, during the joint training, the parameters of the initial architecture are modified so as to minimize a loss function based on a multimodality matrix, having at least two dimensions, defined from said vectors of medical text features obtained by said first encoder and said vectors of vocal and linguistic features obtained by said second encoder.

Advantageously, the training dataset is multimodal, which allows for an enrichment of the data scale, thus overcoming the current limitation related to the restricted size of datasets in psychiatry. Moreover, jointly training the medical text and vocal and linguistic signals encoders on a multimodal training dataset (comprising both textual and vocal data) allows for better capturing the correlations between different types of data. By optimizing the parameters of the initial architecture to minimize a loss function based on a multimodality matrix, the present invention proposes a way to maximize the use of available data, even when it is limited in volume, for better prediction of symptoms and treatments. By integrating additional clinical information into the training data (beyond simple structured labels), the invention overcomes the poor labeling of training vocal data (i.e., vocal signals) by providing a rich context from which the initial architecture can learn. This compensates for the lack of well-annotated vocal data with a more comprehensive and contextual approach to the available data. Additionally, the proposed training, being weakly supervised, offers robustness against the unequal availability of clinical data among different patients.

In other words, the at least one processor is configured to generate said artificial intelligence system for monitoring by jointly training said first encoder and said second encoder of said initial architecture based on said training dataset, the training using:
- as input to the first encoder, messages summarizing the clinical situation of said patient obtained by the biomedical language model receiving as input at least part of the clinical information of said training dataset;
- a multimodality matrix, having at least two dimensions, defined from said vector of medical text features of said first encoder and said vector of vocal and linguistic features of said second encoder.

Jointly training the first and second encoders advantageously allows the initial architecture to learn the unknown links in advance between the patient's clinical data and their vocal signals.

According to other advantageous aspects of the invention, the device comprises one or more of the features described in the following embodiments, taken alone or in any possible combination.

In one embodiment, the generated artificial intelligence system is configured to receive as input at least one message summarizing the clinical situation and at least one vocal signal of a patient, to obtain a matching score for each pair formed between the vector of vocal and linguistic features obtained by the second encoder and each of said at least one vector of medical text features obtained by the first encoder, and to use said at least one matching score to generate said at least one prediction of symptoms, a dosage of at least one treatment prescribed to said patient, and/or transcriptions.

In one embodiment, said device for generating an artificial intelligence system for monitoring a patient further comprises:
- each training sample further comprises, for at least one subject of said plurality of subjects, at least one message from a caregiver comprising at least one characteristic of the speech and/or information related to a speech disorder of said subject, and
- said initial architecture further comprises a third text encoder configured to receive as input at least one message from a caregiver comprising at least one characteristic of the speech and/or information related to a speech disorder of said patient, and to generate, as output, a vector of text features, and
- the at least one processor is further configured to jointly train said first encoder, said second encoder, and said third encoder of said initial architecture, the training using a multimodality matrix having at least three dimensions defined from the vector of medical text features of said first encoder, the vector of vocal and linguistic features of said second encoder, and the vector of text features of said third encoder.

In one embodiment, the training of said initial architecture based on said training dataset comprises, for each training sample, a selection of a part of the training sample to train the initial architecture. Said selection may notably comprise a random selection of the clinical information related to the subject. This random selection of a part only of the clinical information advantageously makes the artificial intelligence system (trained) robust against missing data in the patient's clinical records.

In one embodiment, the demographic data comprises at least: an age, a sex, a weight, a place of residence, and/or a place of birth.

In one embodiment, said prescription concerns at least one among: typical or atypical antipsychotic molecules prescribed in a treatment of schizophrenia or a sedative antipsychotic, a mood stabilizer, an antiepileptic, a hypnotic, an anticholinergic, and/or benzodiazepines/anxiolytics.

In one embodiment, said message summarizing the clinical situation of said patient comprises at least one of the following information: demographic data, a prescription of at least one treatment, responses to at least one psychiatric questionnaire filled out by said patient, responses to at least one psychiatric questionnaire filled out by a caregiver, at least one concentration of a treatment previously measured from a blood sample of said patient (i.e., a blood test of psychotropic drugs) or said prescription of at least one treatment.

In one embodiment, said artificial intelligence system for monitoring is further configured to provide as output a prediction of a symptomatic state of said patient in psychiatry. The term *symptomatic state* is intended to mean a condition of the patient characterized by the presence, severity, or progression of one or more psychiatric symptoms. Such symptoms may include, without limitation, depressive symptoms (e.g., sadness, anhedonia, fatigue), anxiety symptoms (e.g., worry, agitation, panic), sleep disturbances (e.g., insomnia, hypersomnia), cognitive impairments (e.g., memory loss, attention deficit), psychotic manifestations (e.g., hallucinations, delusions), or behavioral anomalies (e.g., withdrawal, aggression). A symptomatic state may correspond to a specific diagnostic category, a phase of a psychiatric disorder, a level of severity measured by a clinical or psychometric score, or a clinically meaningful change in the patient's condition.

In one embodiment, the artificial intelligence system for monitoring is further configured to provide as output the demographic data.

In one embodiment, the artificial intelligence system for monitoring is further configured to provide as output a prediction of at least one indicator, said indicator being at least one among: a blood concentration of an antipsychotic and/or a sedative antipsychotic, a D2 occupancy rate, a presence or absence of benzodiazepines/anxiolytics, a presence or absence of anticholinergics/antidepressants/ mood stabilizers/hypnotics, presence or absence of mood stabilizers, presence or absence of hypnotics/soporifics.

According to one embodiment, said generated artificial intelligence system is configured to receive as input at least one message summarizing the clinical situation and at least one vocal signal of a patient, to obtain a matching score for each pair formed between the vector of vocal and linguistic features obtained by the second encoder and each of said at least one vector of medical text features obtained by the first encoder, and to use said at least one matching score to obtain said at least one prediction of symptoms, a dosage of at least one treatment prescribed to said patient, and/or transcriptions.

In one embodiment, said pretrained foundation model is based on at least one architecture among: a ResNet-type architecture, a Hubert-type architecture, a Whisper-type architecture, a Transformer-type architecture.

The invention also relates to a device for monitoring a patient suffering from psychiatric disorders using said artificial intelligence system for monitoring a patient suffering from psychiatric disorders obtained with the device for generating an artificial intelligence system according to any one of the embodiments, said device for monitoring a patient suffering from psychiatric disorders comprising:
- at least one input configured to receive:
   ∘ clinical information related to said patient comprising at least: a result of a psychiatric questionnaire, at least one prescription of at least one treatment, at least one concentration of a treatment previously measured from a blood sample, and/or demographic data;
   ∘ at least one vocal signal of said patient;
- at least one processor configured to:
   ∘ provide as input to said artificial intelligence system said clinical information related to said patient and said at least one vocal signal of said patient, and obtain as output at least one prediction of symptoms, a dosage of at least one treatment prescribed to said patient, and/or transcriptions;
- at least one output configured to provide said at least one prediction of symptoms, a dosage of at least one treatment prescribed to said patient, and/or transcriptions.

According to one embodiment, said message summarizing the clinical situation of said patient is obtained by providing clinical information related to said patient as input to a pretrained biomedical language model, obtaining, as output, said message summarizing the clinical situation of said patient, said clinical information related to said patient comprising at least: a result of a psychiatric questionnaire, at least one prescription of at least one treatment, at least one concentration of a treatment previously measured from a blood sample, a prescription and/or demographic data.

According to one embodiment, said message summarizing the clinical situation of said patient is entered by a user.

According to one embodiment, obtaining as output at least one prediction of symptoms, a dosage of at least one treatment prescribed to said patient, and/or transcriptions comprises:
- for each of the at least one message summarizing the clinical situation of said patient, obtaining a medical text feature vector by the first medical text encoder of said artificial intelligence system, said first medical text encoder receiving as input said message summarizing the clinical situation of said patient;
- obtaining a vocal and linguistic feature vector by the second vocal and linguistic signal encoder of said artificial intelligence system, said second vocal and linguistic signal encoder receiving as input said at least one vocal signal of said patient;
- for each pair formed between the vocal and linguistic feature vector and each of said at least one medical text feature vector, obtaining a matching score;
- using said at least one matching score to obtain said at least one prediction of symptoms, a dosage of at least one treatment prescribed to said patient, and/or transcriptions.

Another aspect of the invention concerns a computer-implemented method (i.e., a process) for generating an artificial intelligence system for monitoring a patient suffering from psychiatric disorders, said method comprising:
- receiving:
   ∘ a training dataset, comprising a plurality of training samples for a plurality of subjects, each training sample of said dataset comprising, for each subject of said plurality of subjects:
      ▪ a vocal signal of said subject;
      ▪ clinical information related to said subject comprising at least: a result of a psychiatric questionnaire, at least one prescription of at least one treatment, at least one concentration of a treatment previously measured from a blood sample, and/or demographic data;
      ▪ a pretrained biomedical language model (LLM) configured to receive as input, for a patient, clinical information related to said patient comprising at least: a result of a psychiatric questionnaire, at least one prescription of at least one treatment, at least one concentration of a treatment previously measured from a blood sample, and/or demographic data; said biomedical language model being further configured to generate as output a message summarizing the clinical situation of said patient;
   ∘ an initial architecture of said artificial intelligence system for monitoring, said initial architecture being configured to provide at least one prediction of symptoms, a dosage of at least one treatment prescribed to a patient in psychiatry, and/or transcriptions, said initial architecture comprising:
      ▪ a first medical text encoder configured to receive as input a message summarizing the clinical situation of said patient generated by said biomedical language model, and generate as output a vector of medical text features;
      ▪ a second encoder of vocal and linguistic signals configured to receive as input at least one vocal signal of a patient and generate as output a vector of vocal and linguistic features, said second encoder being obtained by a pretrained foundation model;
- generating said artificial intelligence system for monitoring by jointly training said first encoder and said second encoder of said initial architecture based on said training dataset, wherein the joint training comprises iterating until convergence, for each training sample, the following steps:
   ∘ obtaining a message summarizing the clinical situation of said patient by said biomedical language model receiving as input at least part of the clinical information comprised in the training sample;
   ∘ obtaining a vector of medical text features by said first medical text encoder receiving as input the message summarizing the clinical situation;
   ∘ obtaining a vector of vocal and linguistic features by said second encoder of vocal and linguistic signals receiving as input the at least one vocal signal comprised in the training sample;
   ∘ modifying the parameters of the initial architecture so as to minimize a loss function based on a multimodality matrix, having at least two dimensions, defined from said vector of medical text features of said first encoder and said vector of vocal and linguistic features of said second encoder;
- providing as output said artificial intelligence system for monitoring.

Another aspect of the invention concerns a computer-implemented method (i.e., a process) for monitoring a patient suffering from psychiatric disorders using said artificial intelligence system, said method comprising:
- receiving a message summarizing the clinical situation of said patient and at least one vocal signal of said patient;
- providing as input to said artificial intelligence system said message summarizing the clinical situation of said patient and said at least one vocal signal of said patient, and obtaining as output at least one prediction of symptoms, a dosage of at least one treatment prescribed to said patient, and/or transcriptions;
- providing said at least one prediction of symptoms, a dosage of at least one treatment prescribed to said patient, and/or transcriptions.

Moreover, the present disclosure concerns a computer program comprising instructions which, when the program is executed by a computer, cause the computer to carry out a method for generating an artificial intelligence system or a method for monitoring a patient according to any one of the above embodiments when the program is executed by a processor.

The present disclosure also concerns a non-transitory computer-readable storage medium comprising instructions which, when executed by a computer, cause the computer to carry out a method for generating an artificial intelligence system or a method for monitoring a patient, according to the present disclosure.

Such a non-transitory storage device can be, without limitation, an electronic, magnetic, optical, electromagnetic, infrared, or semiconductor device, or any appropriate combination thereof. It should be noted that the following, while providing more specific examples, is simply an illustrative and not exhaustive list, as easily understood by a person skilled in the art: a computer portable floppy disk, a hard disk, a ROM, an EPROM (Electrically-Erasable Programmable Read-Only Memory) or a Flash memory, a CD-ROM portable (Compact-Disc ROM).

### DEFINITIONS

In the present invention, the terms below are defined as follows:

The terms "adapted" and "configured" are used in the present disclosure in a broad sense encompassing the initial configuration, subsequent adaptation or supplementation of the present devices, or any combination thereof, whether by hardware or software means (including firmware).

The term "processor" should not be interpreted as being restricted to hardware capable of executing software, and generally refers to a processing device, which may for example include a computer, a microprocessor, an integrated circuit or a programmable logic device (PLD). The processor may also include one or more graphics processing units (GPUs), whether used for computer graphics and image processing or other functions. Moreover, the instructions and/or data enabling the realization of associated and/or resulting functionalities may be stored on any processor-readable medium such as an integrated circuit, a hard disk, a CD (Compact Disc), an optical disc such as a DVD (Digital Versatile Disc), a RAM (random access memory) or a ROM (read-only memory). The instructions may be stored in particular in hardware, software, firmware or any combination thereof.

The term "Machine Learning (ML)" traditionally designates the computer algorithms that automatically improve through experience, based on training data enabling the adjustment of the parameters of computer models by reducing the gaps between the expected outputs extracted from the training data and the evaluated outputs calculated by the computer models.

### BRIEF DESCRIPTION OF THE FIGURES

The present invention will be better understood, and other specific and nonlimiting aspects of the disclosure will become apparent from the following description of particular embodiments, the description making reference to the appended drawings in which:
**Figure 1** is a functional diagram schematically representing a particular mode of a device for generating an artificial intelligence system for monitoring a patient suffering from psychiatric disorders, in accordance with the present disclosure;
**Figure 2A** and **2B** are functional diagrams schematically representing a particular embodiment of devices for monitoring a patient suffering from psychiatric disorders using the artificial intelligence system for monitoring a patient suffering from psychiatric disorders obtained with the device of Figure 1 according to any one of the embodiments;
**Figure 3** is a flowchart showing the successive steps executed with the device of **Figure 1****;**
**Figure 4** is a flowchart showing the successive steps executed with the device for monitoring a patient suffering from psychiatric disorders of Figure 2;

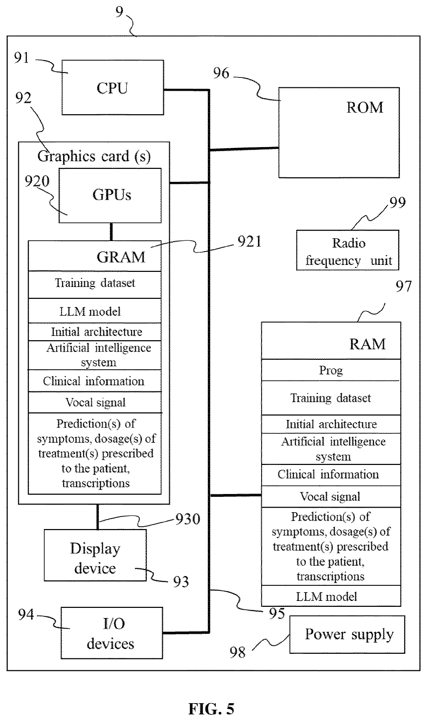

**Figure 5** schematically represents an apparatus integrating the functions of the device(s) of **Figure 1****,** **Figure 2A** and/or **2B;**
**Figure 6** represents the training of the initial architecture based on a two-dimensional multimodality matrix according to one embodiment of the invention;
**Figure 7** represents the training of the initial architecture based on a three-dimensional multimodality matrix according to one embodiment of the invention;
**Figure 8** represents a first example of an embodiment of the artificial intelligence system for inference;
**Figure 9** represents a second example of an embodiment of the artificial intelligence system for inference.

In the figures, the drawings are not to scale and the identical or similar elements are designated by the same references.

### DETAILED DESCRIPTION

The present description illustrates the principles of the present disclosure. It will thus be appreciated that persons skilled in the art will be able to design various arrangements that, although not explicitly described or illustrated herein, embody the principles of the disclosure and are included within its scope.

All examples and conditional language mentioned herein are intended for educational purposes to help the reader understand the principles of the disclosure and the concepts contributed by the inventor(s) to furthering the state of the art, and are to be interpreted as not being limiting to the examples and conditions specifically mentioned.

Moreover, all statements herein reciting principles, aspects, and embodiments of the disclosure, as well as specific examples thereof, are intended to encompass both structural and functional equivalents thereof. Moreover, it is intended that such equivalents include both equivalents that are currently known and equivalents that will be developed in the future, i.e., any element developed that performs the same function, regardless of the structure.

Thus, for example, it will be appreciated by those skilled in the art that the block diagrams presented herein can represent conceptual views of illustrative circuitry embodying the principles of the disclosure. Similarly, it will be appreciated that any flowcharts, state transition diagrams, pseudocode, and the like represent various processes that may be substantially represented in computer-readable media and so executed by a computer or processor, whether or not such computer or processor is explicitly shown.

The functions of the various elements illustrated in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements illustrated in the figures can be implemented in various forms of hardware, software, or combinations thereof. Preferably, these elements are implemented in a combination of hardware and software on one or more appropriately programmed general-purpose devices that may include a processor, a memory, and input/output interfaces.

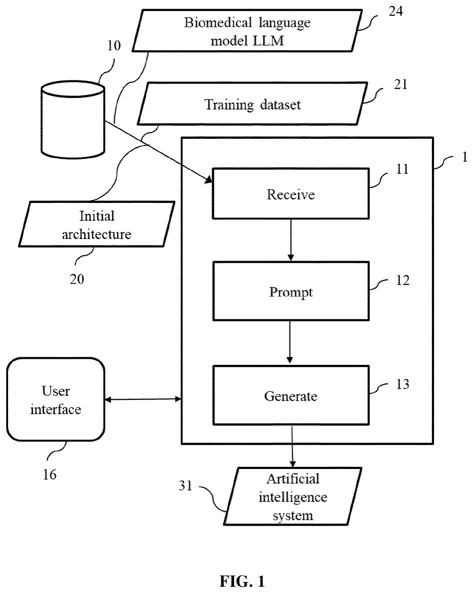

The present disclosure will be described with reference to a particular functional embodiment of the device 1 for generating an artificial intelligence system 31 for monitoring a patient suffering from psychiatric disorders (i.e., training device), as illustrated in **Figure 1****.**

The device 1 is adapted to provide a trained artificial intelligence system 31 for monitoring a patient suffering from psychiatric disorders.

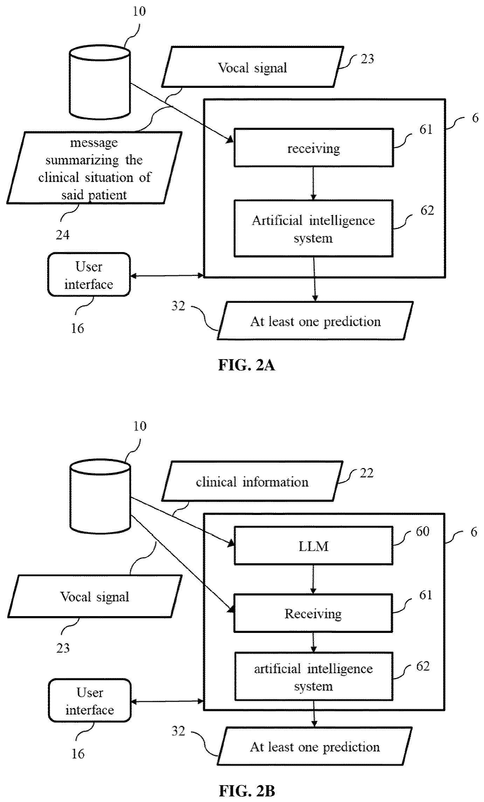

A device 6 for monitoring a patient suffering from psychiatric disorders (i.e., prediction or inference device) is associated with the training device 1 adapted to correctly configure the parameters of the artificial intelligence system 31 of the device 6, represented in **Figure 2****,** which will be described later.

Although the devices 1 and 6 described herein are versatile and equipped with several functions that can be executed alternately or cumulatively, other implementations within the scope of the present disclosure include devices having only some of the functionalities present.

Each of the devices 1 and 6 is advantageously an apparatus, or a physical part of an apparatus, designed, configured, and/or adapted to perform the functions mentioned and produce the effects or results mentioned. In alternative implementations, the device 1 and the device 6 may consist of a set of apparatuses or physical parts of apparatuses, whether grouped in the same machine or in different, possibly remote machines. The device 1 and/or the device 6 may, for example, have distributed functions on a cloud infrastructure and be available to users as a cloud-based service, or have remote functions accessible via an API.

In the following, the modules should be understood as functional entities rather than physically distinct hardware components. They can therefore be integrated either together in the same tangible and concrete component, or distributed in several of these components. Moreover, each of these modules can optionally be shared between at least two physical components. Furthermore, the modules are implemented in hardware, software, firmware, or any combination thereof. They are preferably integrated into at least one processor of the device 1 or the device 6.

The device 1 comprises a module 11 for receiving a training dataset 21, a biomedical language model (LLM) 24, and the initial architecture 20 stored in one or more local or remote databases 10. The latter can take the form of storage resources available from any appropriate storage means, which can in particular be a RAM or an EEPROM (Electrically-Erasable Programmable Read-Only Memory) such as a Flash memory, possibly in an SSD (solid-state drive).

More particularly, the training dataset 21 may comprise a plurality of training samples obtained from a plurality of subjects. Each training sample for each of the subjects may comprise at least one vocal signal of the subject from the plurality of subjects (for example, a vocal signal of at least 200 milliseconds), and optionally an identity of the subject. The vocal signal of the subject can be obtained by a voice recording via a smartphone or a computer equipped with a microphone, by a digital recorder, via a connected or associated medical device, or through a voice recognition device integrated into an application. In other examples, a videoconferencing system or an intelligent voice assistant (such as Amazon Alexa, Google Assistant, or Apple Siri) can also be used to obtain the vocal signal of the subject. Moreover, a portable dictaphone, a tablet with voice recording function, a headset with integrated microphone also offer alternatives for obtaining the vocal signal. The vocal signals can be timestamped. For example, a vocal signal can be recorded in various formats such as WAV, MP3, AAC, FLAC, or CAF, with a minimum sampling frequency of 4 kHz; this allows the portability of the vocal signal on various devices (e.g., personal device, medical instrument in the hospital).

Each training sample may further comprise, for each subject, at least one additional information being clinical information in text format. In other words, each training sample comprises a pair consisting of the vocal signal and the clinical information of the subject (i.e., text).

Each training sample may comprise clinical information related to the subject.

The clinical information may comprise a result of at least one psychiatric questionnaire. A psychiatric questionnaire may, for example, be the Beck Depression Inventory (BDI) to assess the severity of depressive symptoms, the Beck Anxiety Inventory (BAI) to measure the severity of anxiety, or the Young Mania Rating Scale (YMRS) to assess manic symptoms. Any validated questionnaire (Scale) in psychiatry can be used within the scope of the invention. These questionnaires allow clinicians to collect structured data on the psychiatric symptoms of the patient/subject.

The clinical information may further comprise at least one prescription of at least one treatment. The treatment may be: an antidepressant, such as fluoxetine (Prozac) and sertraline (Zoloft), used to treat major depressive disorder and anxiety disorders; an anxiolytic such as lorazepam (Ativan) and alprazolam (Xanax), which help reduce anxiety; an antipsychotic such as risperidone (Risperdal) and quetiapine (Seroquel), prescribed for psychotic disorders such as schizophrenia; mood stabilizers such as lithium and valproate, used in the treatment of bipolar disorders. The at least one prescription may concern at least one molecule among typical or atypical antipsychotic molecules prescribed in the treatment of schizophrenia (for example, aripiprazole (Abilify), risperidone/paliperidone (Risperdal), and olanzapine (Zyprexa)), or sedative antipsychotics (for example, chlorpromazine). The prescription may also include a mood stabilizer such as lithium salts (Teralithe). The prescription of a treatment may comprise information concerning a treatment that does not target a psychiatric disorder of the patient, such as benzodiazepines, anti-epileptics, or anticholinergics, which may have effects on the voice of the subject (e.g., tremor, xerostomia, sedation).

The clinical information may further comprise at least one concentration of a treatment previously measured from a blood sample of the subject. Advantageously, the concentration(s) of a treatment(s), previously measured from a blood sample of the subject, allow obtaining ground truth on adherence to at least part of the prescription.

The clinical information may further comprise demographic data such as the age, the sex, the weight, the place of residence, and/or the place of birth of the subject.

Advantageously, the training dataset 21 is weakly labeled, which allows using training samples that do not all comprise the same clinical information. For example, a training sample may comprise only a vocal signal of the subject and a result of a psychiatric questionnaire. In another example, a training sample may comprise only a vocal signal of the subject and demographic data. In another case, a training sample may comprise a vocal signal of the subject, a prescription of a treatment, and a concentration of a treatment previously measured from a blood sample. Using a weakly labeled training dataset 21 further allows the artificial intelligence system 31 for monitoring to learn relationships, between the data of the training dataset 21, not known in advance.

In one embodiment, the module 11 is further configured to receive a pretraining dataset comprising data from one or more public databases (e.g., audio, description) and/or one or more private/proprietary databases. The public databases used to pretrain allow capturing (1) the variations in speech, such as emotions (for example, CREMA-D, RAVDESS, Music emotion datasets), the emotions being linked to psychiatric disorders; (2) sound events that enrich the model and make it robust to various noises and recording conditions (for example, ESC50, FSD50K, US8K, DCASE17 Task 4, AudioSet). AudioSet, in particular, provides an abundance of data on human speech in natural conditions, with very rich and detailed labels. It is possible that the pretraining data set comprises data from one of the databases mentioned or a mixture of these databases.

In one embodiment, the training dataset 21 comprises, among others, non-specific training samples (i.e., non-specific to psychiatric disorders), each comprising a pair of data vocal signal of a subject and information related to the subject and/or the vocal signal, obtained from at least one of these public databases. The information related to the subject and/or the vocal signal may or may not include clinical information concerning said subject. Adding non-specific training samples allows obtaining better results during the training of the artificial intelligence system 31. According to this embodiment, the use of pretrained encoders is possible but not necessary. Large language models (LLM) are advanced neural networks designed to understand and generate text. In the present disclosure, the biomedical language model 24 (LLM) may be configured to receive, as input, for a patient, clinical information related to said patient comprising at least: a result of a psychiatric questionnaire, at least one prescription of at least one treatment, at least one concentration of a treatment previously measured from a blood sample, and/or demographic data; the biomedical language model 24 being further configured to generate, as output, a message 25 summarizing the clinical situation of the patient.

The biomedical language model 24 (LLM) may be a model pretrained on a generic language database and based on the Transformer architecture such as a BERT-type model (Bidirectional Encoder Representations from Transformers), T5 (Text-To-Text Transfer Transformer), Mistral-type, or Mixtral-type. The biomedical language model 24 (LLM) may, for example, be of the Mistral 7B, GEMMA 9B, or BIOMISTRAL type.

The message 25 summarizing the clinical situation of the patient (output of the LLM 24) may be configured to comprise at least one of the following information: demographic data, a prescription of at least one treatment, responses to at least one psychiatric questionnaire filled out by the patient, responses to at least one psychiatric questionnaire filled out by a caregiver, at least one concentration of a treatment previously measured from a blood sample of the patient (i.e., a blood test of psychotropic drugs). Advantageously, the message 25 summarizing the clinical situation allows obtaining a short text that provides a summary of the necessary information for the analysis of the medical situation by the first encoder.

In one embodiment, the initial architecture 20 of the artificial intelligence system 31 comprises at least: a first medical text encoder and a second encoder of vocal and linguistic signals. In the present description, by initial architecture it is meant the set consisting of: the first medical text encoder (pretrained) and the second encoder of vocal and linguistic signals (pretrained) before the training using the training dataset 21. In particular, the first medical text encoder (pretrained) and the second encoder of vocal and linguistic signals (pretrained) can be pre-trained using the pretraining dataset.

The first medical text encoder may be configured to receive, as input, a message 25 summarizing the clinical situation of the patient, generated by the biomedical language model 24 (LLM), and generate, as output, a vector of medical text features. The first medical text encoder may be based on a pretrained Transformer-type architecture that can be further fine-tuned using a medical text database, notably comprising text in reference to psychiatric disorders. A GPT (Generative Pre-trained Transformer), BERT (Bidirectional Encoder Representations from Transformers), T5, Mistral-type, or Mixtral-type architecture can be used for the first encoder.

The second encoder of vocal and linguistic signals may be configured to receive, as input, at least one vocal signal of a patient and generate, as output, a vector of vocal and linguistic features. This second encoder may be obtained by a pretrained foundation model (foundational model). The pretrained foundation model may be based on a ResNet-type architecture or a Transformer-type architecture. In one example, the pretrained foundation model is a wav2vec 2.0, hubertXL, or Whisper model designed to efficiently and accurately convert audio inputs into text, even with limited amounts of annotated data. This wav2vec 2.0, hubertXL, or Whisper model, which uses a Transformer-type architecture, is pretrained with large amounts of unannotated audio data. This allows the wav2vec 2.0, hubertXL, or Whisper model to learn useful representations of audio features without needing text transcriptions.

In one embodiment, the initial architecture 20 further comprises a third text encoder configured to receive, as input, at least one message from a caregiver comprising at least one characteristic of the speech and/or information related to a speech disorder of the patient, and to generate, as output, a vector of text features (latent vector). A GPT, BERT, T5, Mistral, or Mixtral architecture can also be used for the third encoder.

In this embodiment, each training sample of the training dataset 21 further comprises at least one message from a caregiver (e.g., doctor, nurse, etc.) comprising at least one characteristic of the speech and/or an information related to a speech disorder of the subject. A message from a caregiver may be a written text or a vocal signal comprising words forming an intelligible message.

The device 1 may further comprise a module 12 configured for prompting the LLM model 24 with specific examples to obtain more precise answers. For example, the module 12 may adopt an innovative approach to creating prompts to enable the generation of medical texts adapted to the specific needs of psychiatry. This approach combines medical expertise and advanced linguistic modeling techniques. It integrates contextual learning (in-context learning), which consists of using precise clinical examples to provide relevant medical context. Moreover, it uses the « chain-of-thought » technique, which breaks down the text generation process into a series of logical and sequential steps. This approach allows building more coherent and nuanced prompts, faithfully reflecting the subtleties of psychiatric situations, and thus improving the quality and relevance of the texts generated by the biomedical LLM model 24.

For example, models such as GEMMA-2-9b-it and BIOMISTRAL-7B can be used with carefully formulated prompts based on templates. Several models have been used depending on the task to be accomplished, in order to determine those that gave the best results. A particularly effective technique is contextual learning.

In one example, the model for prompting the LLM 24 may comprise a first part of text capable of asking the LLM model 24 to provide a detailed textual description of a patient based on the clinical data provided, this description being short with complete sentences with the aim of obtaining an overview of the patient's history and current clinical status. Then, the model for prompting may comprise clinical data including demographic data (e.g., age, sex, mother tongue, education level, place of birth), as well as results (i.e., scores) of psychiatric questionnaires obtained from questionnaires such as the BDI, the PHQ-9 (Patient Health Questionnaire-9), the GAD-7 (Generalized Anxiety Disorder-7), the AIS (Athens Insomnia Scale), the IADL (Instrumental Activities of Daily Living), the MoCA (Montreal Cognitive Assessment). The model for prompting may further comprise the results (i.e., scores) of psychiatric questionnaires provided by a healthcare professional such as a psychiatrist. In another example, the results of psychiatric questionnaires may be obtained via self-assessment by the patient.

In another example, the model for prompting may comprise for a first patient the demographic data mentioned above, the results of psychiatric questionnaires plus a description of the first patient in the form of a message 25 summarizing the clinical situation of the patient: « This 29-year-old woman from Paris suffers from severe depression and high anxiety, with serious sleep disturbances. Despite relatively preserved cognitive function, she occasionally needs help with her daily tasks.». This same model for prompting may further comprise the same information for at least a second patient, and optionally N other patients.

In one embodiment, the model for prompting may focus on an answer of a particular question from a questionnaire, for example when the model for prompting comprises a particular clinical data such as a score from a psychiatric questionnaire, in addition to the clinical data. In one example, the score may be that of question G12 of the PANSS questionnaire (Positive and Negative Syndrome Scale), filled out by a psychiatrist, the objective being to complete the message 25 summarizing the clinical situation of the patient based on the score related to the question « lack of judgment and insight » of the PANSS questionnaire, a scale used to assess the awareness of patients regarding their psychiatric state and their ability to understand their need for treatment. The message 25 summarizing the clinical situation of the patient may vary depending on the score: for example, a score of 4 is interpreted as indicating moderate impairment in judgment and insight, in this case the message 25 summarizing the clinical situation of the patient included in the model for prompting may be: « The patient is a 33-year-old person from Sydney, Australia, who has completed higher education but did not obtain a diploma. The patient scores 4 on the PANSS scale, indicating moderate lack of judgment and insight. This suggests notable difficulty in recognizing their psychiatric state and understanding the need for treatment, which may affect their decision-making and planning.». In another example, a score of 6, on the other hand, reflects severe impairment, leading to a message such as: « The patient is a 38-year-old man from Berlin, Germany, who completed professional training. His mother tongue is Spanish. The patient scores 6 on the PANSS scale, indicating severe deficiency in judgment and insight. He has great difficulty recognizing his psychiatric state, probably denies the need for treatment, and experiences great difficulty making decisions and developing realistic plans. This lack of insight is very concerning and requires immediate attention in his care plan.».

The biomedical language model 24 (LLM) used demonstrates in this case its ability to generate a coherent message 25 summarizing the clinical situation of the patient that aligns with the degree of judgment and insight of the patient, and adjusted according to the PANSS score, which shows a nuanced understanding of the scale and the associated clinical needs. The device 1 may further comprise a module 13 for generating the artificial intelligence system 31 for monitoring by training the initial architecture 20, notably the first encoder, the second encoder, and optionally the third encoder.

In the embodiment where the initial architecture 20 comprises the first encoder and the second encoder, the module 13 is configured to jointly train, with a contrastive approach, the first encoder and the second encoder of the initial architecture 20, based on the training dataset 21. Notably, the module 13 jointly trains the first encoder and the second encoder to predict the correct pairings of the training samples of the set 21 (pairing between vocal signal and text i.e., clinical information of the subject). During testing, the trained first medical text encoder is therefore capable of synthesizing a linear classifier without prior training (zero-shot linear classifier) by incorporating the names or descriptions of the target classes of the training dataset 21. Indeed, contrastive training is a machine learning technique where the model learns to differentiate between pairs of similar and dissimilar data points. The main objective is to bring similar pairs closer together in the embedding space while moving dissimilar pairs further apart. In the case of the present invention, a positive pair can be the vocal signal of the subject and the clinical information of the subject.

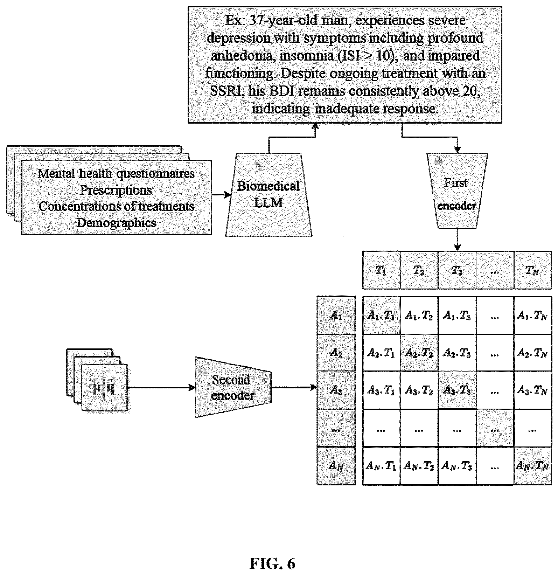

More specifically, the training is based on the use of a multimodality matrix, having at least two dimensions, and defined from the vector of medical text features of the first encoder and the vector of vocal and linguistic features of the second encoder. **Figure 6** represents an illustration of this embodiment with a two-dimensional multimodality matrix. For the embodiment in which the initial architecture comprises the first encoder and the second encoder: given the batch of N pairs (vocal signal, text - i.e., clinical information) included in the training dataset 21, the initial architecture 20 is trained to predict which of the N × N possible pairings (sound, text) within the batch actually occurred. To do this, the initial architecture 20 learns a multimodal embedding space by jointly training the first medical text encoder and the second encoder of vocal and linguistic signals to maximize the cosine similarity of the embeddings of the sounds and texts of the N true pairs in the batch while minimizing the cosine similarity of the embeddings of the N² - N incorrect pairs. In one example, a symmetric cross-entropy loss function is used for optimization on these similarity scores.

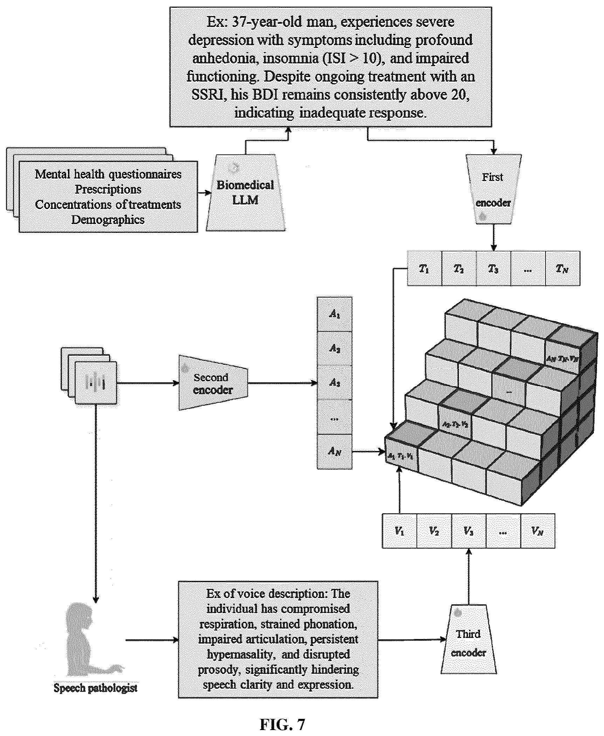

With reference to the embodiment in which the initial architecture comprises the first encoder, the second encoder, and the third encoder, **Figure 7** represents an illustration of the embodiment comprising the use of a three-dimensional multimodality matrix during training.

In this case, the third dimension of the multimodality matrix corresponds to the vector of text features (output of the third encoder); the initial architecture 20 is trained in a similar manner to that described for **Figure 6****,** but with additional complexity related to the integration of this third dimension. In particular, the third encoder allows capturing additional information from the textual data, such as advanced semantic nuances or contextual relationships between biomedical concepts. This additional dimension allows the architecture to model more finely the multimodal relationships between the vocal signal and the text. Thus, during training, the initial architecture 20 is optimized not only to maximize the cosine similarity between the embeddings of the correct pairs (sound, text) within a batch, but also to minimize this similarity for the incorrect pairs, while taking into account the semantic richness introduced by the third encoder. For example, a symmetric cross-entropy loss function can be applied to adjust the weights of the initial architecture 20 based on the similarity scores in this multimodal space.

The device 6, illustrated in Figures 2A and 2B, is further adapted to monitor a patient suffering from psychiatric disorders using the artificial intelligence system 31 for monitoring a patient suffering from psychiatric disorders obtained with the device 1.

The device 6 may comprise an input module 61 (e.g., an input) configured to receive a message 25 summarizing the clinical situation of said patient, for example entered by a user (i.e., healthcare professional) or selected by the user from a predefined list of command/request prompts. The at least one input of the device 6 may be further configured to receive at least one vocal signal 23 of the patient.

The message summarizing the clinical situation of said patient 25, as the message obtained by the LLM 24, is an unstructured or semi-structured textual data expressed in natural language, conveying all or part of the information relating to the patient's health status, demographic profile, medical history, behavior, or functional abilities. Such a message may, without limitation, include demographic data (age, sex, gender, place of birth, native language, level of education), medical or psychiatric diagnoses, clinical and psychometric results, indicators or scores, symptoms and their severity (e.g., depression, anxiety, sleep disorders, cognitive disorders), information on functional autonomy and the ability to perform activities of daily living, data relating to treatment adherence, as well as any other relevant clinical observation. In one example, the message 25 comprises: (i) demographic data including at least the patient's age and sex or gender, (ii) at least one medical or psychiatric diagnosis, and (iii) at least one piece of information relating to a clinical or psychometric indicator, a symptom and its severity, or the patient's functional autonomy.

The device 6 may further comprise a module 62 (e.g., at least one processor) configured to provide as input to the artificial intelligence system 31 obtained by the device 1 the clinical information related to the patient (e.g., message summarizing the clinical situation of said patient 25) and the at least one vocal signal 23 of the patient, and obtain, as output, at least one prediction of symptoms, a dosage of at least one treatment prescribed to the patient, and/or transcriptions 32. In other words, the module 62 is configured to use the artificial intelligence system 31 for inference.

In more detail, the module 62 is configured to provide, as input to the first text encoder, each of said at least one message summarizing the clinical situation of the patient 25, so as to obtain a medical text feature vector for each message 25. The module 62 is also configured to provide, subsequently or in parallel, as input to the second vocal and linguistic signal encoder, said at least one vocal signal, so as to obtain a vocal and linguistic feature vector. A correspondence score is then calculated for each pair formed between the vocal and linguistic feature vector and each of said at least one medical text feature vector. The correspondence score(s) are used to obtain said at least one prediction of symptoms, prediction of a dosage of at least one treatment prescribed to the patient, and/or transcripts.

In one embodiment, at least two messages summarizing the clinical situation of said patient are received as input.

In one embodiment, the artificial intelligence system 31 is configured to receive as input at least two messages summarizing the clinical situation of said patient. These messages may be entered by the user or obtained by means of a prompt using an initial message and a set of candidate textual variants. In this embodiment, a correspondence score is calculated for each pair formed between the vocal and linguistic feature vector obtained by the second encoder and each of the at least two medical text feature vectors obtained by the first encoder. The compatibility scores produced for each variant may then be normalized, for example by a softmax function, to obtain a probability distribution. The message summarizing the clinical situation of said patient (e.g., the textual variant) associated with the highest probability is selected as the output prediction of the artificial intelligence system 31. It will be readily appreciated by those skilled in the art that the same can be performed for three or more input messages.

In one embodiment, the module 62 is configured to operate the artificial intelligence system 31 to operate in zero-shot mode, i.e., without a previously trained fusion model. In this mode, the artificial intelligence system 31 receives as input the message 25 and the at least one vocal signal 23 of the patient. The first text encoder is configured to receive the message 25 together with a set of candidate textual variants, and to generate, for each variant, a multidimensional medical text feature vector. The second encoder is configured to receive the vocal signal and produce an audio and linguistic feature vector. In the absence of a previously trained fusion model, the artificial intelligence system 31 is configured to calculate, for each pair formed of a medical text feature vector and an audio and linguistic feature vector, a similarity measure, such as a cosine similarity, the vectors having been previously normalized. The similarity measures thus obtained may then be scaled using a predefined temperature factor and transformed into relative probabilities by means of a softmax function. The textual variant associated with the highest probability is then selected as the output of the artificial intelligence system 31.

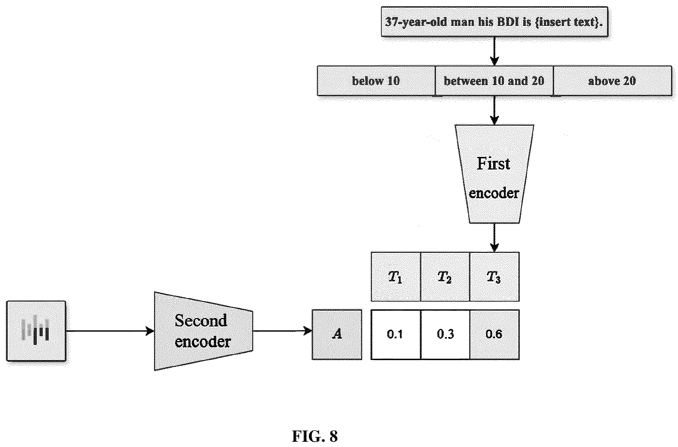

**Figure 8** provides an illustration of the use of the artificial intelligence system 31 for inference as implemented by the device 6. For example, the artificial intelligence system 31 receives, as input, a message 25 summarizing the clinical situation of the patient, the message being: « 37-year-old man, his BDI score is {insert text} ». The BDI being the Beck Depression Inventory. The text to be inserted may, for example, be: « below 10 », « between 10 and 20 » and « above 20 ». The first encoder receives the message and the text insertion options and generates, as output, the vector of medical text features for the three text insertion message options: [T₁, T₂, T₃]. In other words, in this example, three alternative messages are provided as input. On its side, the second encoder receives, as input, a vocal signal of the patient and generates the vector of vocal and linguistic features [A]. Then, the artificial intelligence system 31 combines the two outputs (i.e., [T₁, T₂, T₃] and [A]) so as to predict the probabilities that the BDI of the man is « below 10 », « between 10 and 20 », and « above 20 ». In this example, the final prediction of the artificial intelligence system 31 for this vocal signal of the patient will be « 37-year-old man, his BDI score is above 20 » because the score obtained for T3 is the one with the highest probability (i.e., 0.6). In this example, the texts to be inserted may be "below 10," "between 10 and 20," or "above 20," corresponding respectively to three message variants. The first text encoder receives the message and each of the three variants and generates, for each variant, a multidimensional text feature vector. The second encoder, configured for vocal and linguistic analysis, receives the vocal signal and generates a vocal and linguistic feature vector A. In the absence of a trained fusion model, the artificial intelligence system 31 is configured to calculate, for each pair (Ti, A), a cosine similarity between the text feature vector and the vocal and linguistic feature vector, each having been previously normalized (for example, according to the L2 norm). The similarities (i.e., correspondence scores) thus obtained are then scaled using a predefined temperature and transformed into relative probabilities by means of a softmax function. The message variant having the highest probability is then selected as the output of the artificial intelligence system 31.

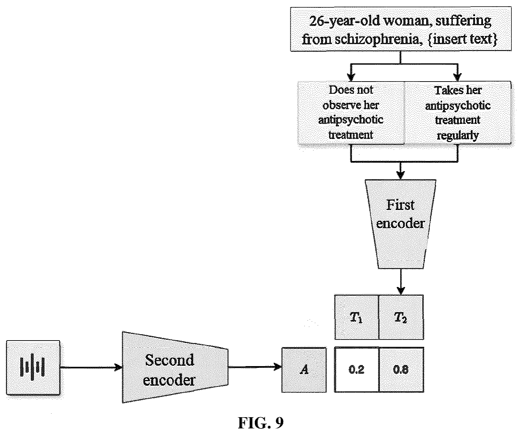

**Figure 9** provides another illustration of the use of the artificial intelligence system 31 for inference as implemented by the device 6. For example, the artificial intelligence system 31 receives, as input, a message 25 summarizing the clinical situation of the patient, the message being: « 26-year-old woman, suffering from schizophrenia, {insert text} ». The text to be inserted may, for example, be: « does not observe her antipsychotic treatment », or « takes her antipsychotic treatment regularly ». The first encoder receives the message and the text insertion options and generates, as output, the vector of medical text features for the two text insertion options: [T₁, T₂]. On its side, the second encoder receives, as input, a vocal signal of the patient and generates the vector of vocal and linguistic features [A]. Then, the artificial intelligence system 31 combines the two outputs (i.e., [T₁, T₂] and [A]) so as to predict the probabilities that the 26-year-old woman suffering from schizophrenia « does not observe her antipsychotic treatment » or « takes her antipsychotic treatment regularly ». In this example, the final prediction of the artificial intelligence system 31 for this vocal signal of the patient will be « 26-year-old woman, suffering from schizophrenia, takes her antipsychotic treatment regularly » because the score obtained for T₂ is the one with the highest probability (i.e., 0.8).

In another embodiment, the module 62 is configured to operate the artificial intelligence system 31 so as to combine the outputs of the first text encoder and the second audio encoder by means of a previously trained fusion head. The first encoder is configured to receive the message 25 and a set of candidate textual variants, and to generate, for each variant, a multidimensional text feature vector. The second encoder is configured to receive the vocal signal 23 and produce a vocal and linguistic feature vector A. The text feature vectors and the vocal and linguistic feature vector A are then provided to the previously trained fusion head. Said fusion head is configured to calculate, for each pair composed of a text feature vector and the vocal and linguistic feature vector A, a compatibility score (i.e., correspondence score), such as a logit, for example by means of a bilinear product or a dot product after projection of the vectors into a common space, or by concatenating the vectors and submitting them to a multilayer perceptron (MLP) with weights shared between the variants, or by applying a multiplicative combination (Hadamard) followed by a linear projection. The compatibility scores produced for each variant may then be normalized by a softmax function to obtain a probability distribution. The textual variant associated with the highest probability is then selected as the output of the artificial intelligence system 31.

In one embodiment, the module 62 is configured to operate the artificial intelligence system 31 so as to evaluate, from a single message 25 and a vocal signal 23, the probability that said message constitutes a correct inference. The first text encoder is configured to receive the message 25 and generate a multidimensional text feature vector. The second encoder is configured to receive the vocal signal 23 and produce a vocal and linguistic feature vector. The two feature vectors thus obtained may then be combined by means of a previously trained fusion head. Said fusion head is configured to calculate a compatibility score (i.e., correspondence score) between the textual content and the vocal and linguistic features, for example by means of a bilinear product or a dot product after projection into a common space, by concatenation followed by a multilayer perceptron (MLP), or by a multiplicative combination (Hadamard) followed by a linear projection. The compatibility score thus calculated may then be transformed, by means of a sigmoid function, into a probability between 0 and 1 indicating the confidence of the artificial intelligence system 31 that the message constitutes a correct inference. A binary decision ("correct" or "incorrect") may then be obtained by comparing said probability with a predefined threshold.

In one embodiment illustrated in figure 2B, the device 6 is further configured to receive and use a pretrained biomedical language model 24 (LLM), such as that described for the device 1. The biomedical language model 24 (LLM) is therefore adapted to generate a message 25 summarizing the clinical situation of said patient from the clinical information 22 related to the patient. Consequently, according to this embodiment the reception module 61 is configured to receive clinical information 22 related to the patient extracted from the medical records, and no longer a message 25 summarizing the clinical situation of said patient entered by a user. The clinical information 22 related to the patient may comprise: a result of a psychiatric questionnaire, at least one prescription of at least one treatment, at least one concentration of a treatment previously measured from a blood sample, and/or demographic data. The device 6 may comprise a module 60 configured to generate a message summarizing the clinical situation of said patient from the clinical information 22 relating to the patient by using the LLM. In this embodiment, the message 25 summarizing the clinical situation of said patient generated by the LLM is therefore provided as input to the first encoder.

The transcriptions offer several advantages by facilitating understanding for a patient with hearing difficulties, for example. Moreover, they allow for quick search and referencing of specific words or phrases, improving indexing and information retrieval. Furthermore, they serve as support for creating medical reports and detailed analyses.

The device 1 and the device 6 interact with a user interface 16, through which information can be entered and retrieved by a user. The user interface 16 comprises any appropriate means for entering or retrieving data, information, or instructions, notably visual, tactile, and/or audio capabilities that may include one or more of the following well-known means to those skilled in the art: a screen, a keyboard, a control ball, a touchpad, a touch screen, a speaker, a voice recognition system; other ways of entering commands such as, for example, voice recognition are also possible.

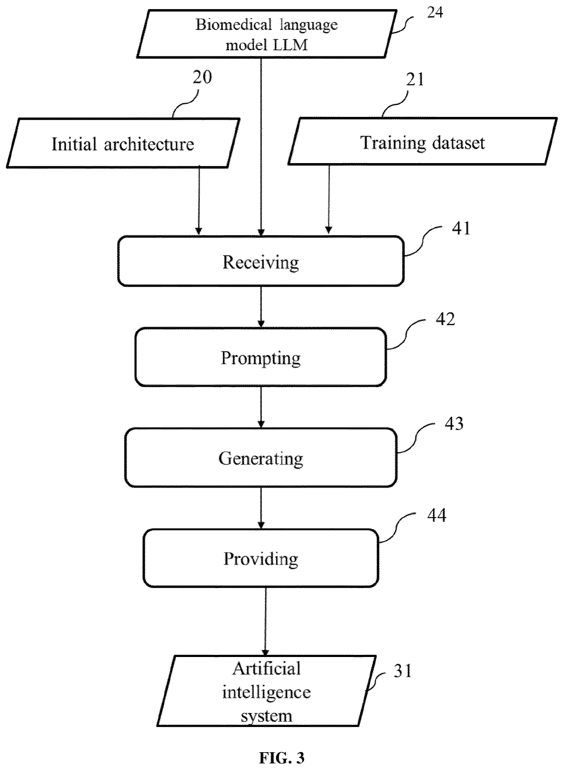

In its automatic actions, the device 1 may, for example, execute the following process (**Figure 3**):
- receiving (step 41):
   ∘ a training dataset 21, comprising a plurality of training samples for a plurality of subjects, each training sample of said dataset 21 comprising, for each subject of said plurality of subjects:
      ▪ a vocal signal of said subject;
      ▪ clinical information related to said subject comprising at least: a result of a psychiatric questionnaire, at least one prescription of at least one treatment, at least one concentration of a treatment previously measured from a blood sample, and/or demographic data;
   ∘ a pretrained biomedical language model 24 (LLM) configured to receive as input, for a patient, clinical information related to said patient comprising at least: a result of a psychiatric questionnaire, at least one prescription of at least one treatment, at least one concentration of a treatment previously measured from a blood sample, and/or demographic data; said biomedical language model 24 being further configured to generate, as output, a message 25 summarizing the clinical situation of said patient;
   ∘ an initial architecture 20 of said artificial intelligence system 31 for monitoring, said initial architecture 20 being configured to provide at least one prediction of symptoms, a dosage of at least one treatment prescribed to a patient in psychiatry, and/or transcriptions, said initial architecture 20 comprising:
      ▪ a first medical text encoder configured to receive, as input, a message 25 summarizing the clinical situation of said patient generated by said biomedical language model, and generate, as output, a vector of medical text features;
      ▪ a second encoder of vocal and linguistic signals configured to receive, as input, at least one vocal signal of a patient and generate, as output, a vector of vocal and linguistic features, said second encoder being obtained by a pretrained foundation model;
- (step 43) generating said artificial intelligence system 31 for monitoring by jointly training said first encoder and the second encoder of the initial architecture 20 based on the training dataset 21, wherein the joint training comprises iterating until convergence, for each training sample, the following steps:
   ∘ obtaining a message 25 summarizing the clinical situation of said patient by said biomedical language model 24 receiving, as input, at least part of the clinical information comprised in the training sample;
   ∘ obtaining a vector of medical text features by said first medical text encoder receiving, as input, the message 25 summarizing the clinical situation;
   ∘ obtaining a vector of vocal and linguistic features by said second encoder of vocal and linguistic signals receiving, as input, the at least one vocal signal comprised in the training sample;
   o modifying the parameters of the initial architecture 20 so as to minimize a loss function based on a multimodality matrix, having at least two dimensions, defined from said vector of medical text features of said first encoder and said vector of vocal and linguistic features of said second encoder;
- providing (step 44), as output, the artificial intelligence system 31 for monitoring.

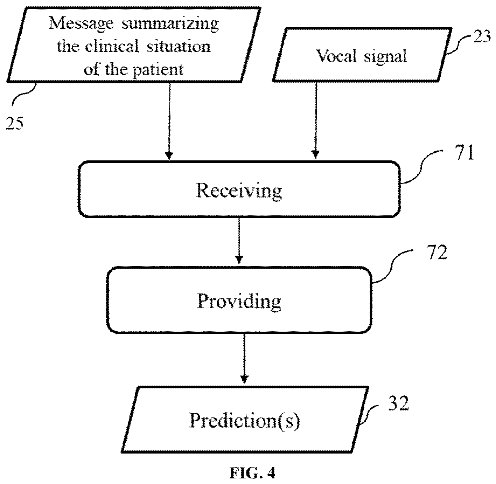

In its automatic actions, the device 6 may, for example, execute the following process for monitoring a patient suffering from psychiatric disorders using said artificial intelligence system 31 (**Figure 4**):
- receiving a message 25 summarizing the clinical situation of said patient and at least one vocal signal 23 of said patient (step 71);
- providing as input to said artificial intelligence system 31 said message 25 summarizing the clinical situation of said patient and said at least one vocal signal of said patient, and obtaining, as output, at least one prediction of symptoms, a dosage of at least one treatment prescribed to said patient, and/or transcriptions (step 72);
- providing said at least one prediction of symptoms, a dosage of at least one treatment prescribed to said patient, and/or transcriptions 32.

In one embodiment, the device 6 and the method (i.e., the process) for monitoring a patient suffering from psychiatric disorders are intended to predict the effectiveness of a treatment administered to the patient. In particular, the treatment is a treatment used in mental health, for example a treatment for depression, schizophrenia, and/or bipolar disorder.

In one embodiment, the device 6 and the method (i.e., the process) for monitoring a patient suffering from psychiatric disorders are intended to determine the patient's adherence to a prescribed treatment. In particular, the treatment is a treatment used in mental health, for example a treatment for depression, schizophrenia, and/or bipolar disorder.

In one embodiment, the device 6 and the method (i.e., the process) for monitoring a patient suffering from psychiatric disorders are intended to estimate the dosage of an antipsychotic treatment taken by the patient, taking into account the patient's age, weight, and sex.

In one embodiment, the device 6 and the method (i.e., the process) for monitoring a patient suffering from psychiatric disorders are intended to determine the evolution over time of the psychiatric disorders from which the patient suffers. The psychiatric disorders may, for example, be depression, in particular severe depression, schizophrenia, and/or bipolar disorder.

In one embodiment, the device 6 and the method (i.e., the process) for monitoring a patient suffering from psychiatric disorders are intended to determine the evolution of the symptoms from which the patient suffers, for example depression, anxiety, fatigue, and/or insomnia.

In one embodiment, the device 6 and the method (i.e., the process) for monitoring a patient suffering from psychiatric disorders are intended to predict the score of a questionnaire used in clinical practice, such as, for example, the MADRS, PHQ-9, GAD-7, AIS, MFI, and/or MCSI questionnaires.

Examples of psychiatric disorders include depression, in particular severe depression, schizophrenia, and/or bipolar disorder.

Examples of treatments used in mental health, in particular examples of treatments for depression, schizophrenia, and/or bipolar disorder, include risperidone, paliperidone, olanzapine, aripiprazole, chlorpromazine, loxapine, and cyamemazine.

Another aspect of the invention concerns a method (i.e., a process) for providing monitoring and/or adapted treatment to a patient suffering from psychiatric disorders, said method comprising:
- monitoring the patient using the device 6 as described above or according to the method as described above, and
- adapting the monitoring and/or the treatment prescribed to the patient.

For example, adapting the monitoring and/or the treatment prescribed to the patient may include increasing the frequency of medical visits, increasing or decreasing the dosage of the treatment prescribed to the patient.

A particular apparatus 9 embodies the device 1 as well as the device 6 described above. It corresponds, for example, to a workstation, a laptop, a tablet, a smartphone, or an augmented reality headset (HMD). This apparatus 9 comprises the following elements, connected to each other by an address and data bus 95 that also transports a clock signal (« clock signal »):
- a microprocessor 91 (or CPU);
- a graphics card 92 comprising several graphics processing units (or GPU) 920 and a graphics RAM (GRAM) 521;
- a non-volatile ROM-type memory 96;
- a RAM-type memory 97;
- one or more input/output (I/O) devices 94 such as, for example, a keyboard, a mouse, a control ball, a touchpad, a touch screen, a speaker, a voice recognition system; other ways of entering commands such as, for example, voice recognition are also possible;
- a power supply 98; and
- a radio frequency unit 99.

In one embodiment, the power supply 98 is external to the apparatus 9.

The apparatus 9 also comprises a display device 93 of the display screen type directly connected to the graphics card 92 to display synthesized images calculated and composed in the graphics card. Using a dedicated bus 930 to connect the display device 93 to the graphics card 92 offers the advantage of having much higher data transmission rates and thus reducing the latency time for displaying the images composed by the graphics card.

In one embodiment, a display device is external to the apparatus 9 and is connected thereto by a cable or wirelessly to transmit the display signals. The apparatus 9, for example via the graphics card 92, comprises a transmission or connection interface adapted to transmit a display signal to an external display means such as, for example, an LCD or plasma screen or a video projector. To this end, the radio frequency unit 99 can be used for wireless transmissions.

It should be noted that the term "register" used below in the description of the memories 97 and 921 can designate in each of the memories mentioned, a memory area of small capacity (a few binary data) as well as a memory area of large capacity (allowing to store an entire program or to calculate or display all or part of the data representative of the data). Similarly, the registers represented for the RAM 97 and the GRAM 921 can be arranged and constituted in any manner, and each of them does not necessarily correspond to adjacent memory locations and can be distributed otherwise (which covers in particular the case where a register comprises several smaller registers).

At power-up, the microprocessor 91 loads and executes the instructions of the program contained in the RAM 97.

As the skilled person will understand, the presence of the graphics card 92 is not mandatory, and can be replaced by complete processing by the central unit and/or simpler implementations of visualization.

In one embodiment, the device 1 can be implemented differently from a standalone software, and an apparatus or a set of apparatuses comprising only parts of the apparatus 9 can be operated via an API call or a cloud interface.

## Claims

1. A device (1) for generating an artificial intelligence system (31) for monitoring a patient suffering from psychiatric disorders, said device (1) comprising:
- at least one input configured to receive (11):
∘ a training dataset (21), comprising a plurality of training samples for a plurality of subjects, each training sample of said dataset (21) comprising, for each subject of said plurality of subjects:
• a vocal signal of said subject;
• clinical information related to said subject comprising at least: a result of a psychiatric questionnaire, at least one prescription of at least one treatment, at least one concentration of a treatment previously measured from a blood sample, and/or demographic data;
∘ a pretrained biomedical language model (24) (LLM) configured to receive, as input, for a patient, clinical information related to said patient comprising at least: a result of a psychiatric questionnaire, at least one prescription of at least one treatment, at least one concentration of a treatment previously measured from a blood sample, and/or demographic data; said biomedical language model (24) being further configured to generate, as output, a message summarizing the clinical situation of said patient (25);
∘ an initial architecture (20) of said artificial intelligence system (31) for monitoring, said initial architecture (20) being configured to provide at least one prediction of symptoms, a dosage of at least one treatment prescribed to a patient in psychiatry, and/or transcriptions, said initial architecture (20) comprising:
▪ a first medical text encoder configured to receive, as input, a message (25) summarizing the clinical situation of said patient generated by said biomedical language model, and generate, as output, a vector of medical text features;
▪ a second encoder of vocal and linguistic signals configured to receive, as input, at least one vocal signal of a patient and generate, as output, a vector of vocal and linguistic features, said second encoder being obtained by a pre-trained foundation model;
- at least one processor configured to generate (13) said artificial intelligence system (31) for monitoring by jointly training said first encoder and said second encoder of said initial architecture (20) based on said training dataset (21), wherein the joint training comprises iterating until convergence, for each training sample, the following steps:
∘ obtaining a message summarizing the clinical situation of said patient (25) by said biomedical language model (24) receiving, as input, at least part of the clinical information comprised in the training sample;
∘ obtaining a vector of medical text features by said first medical text encoder receiving, as input, the message summarizing the clinical situation (25);
∘ obtaining a vector of vocal and linguistic features by said second encoder of vocal and linguistic signals receiving, as input, the at least one vocal signal comprised in the training sample;
∘ modifying the parameters of the initial architecture (20) so as to minimize a loss function based on a multimodality matrix, having at least two dimensions, defined from said vector of medical text features of said first encoder and said vector of vocal and linguistic features of said second encoder;
- at least one output configured to provide as output said artificial intelligence system (31) for monitoring.

2. The device according to claim 1, wherein:
- each training sample further comprises, for at least one subject of said plurality of subjects, at least one message from a caregiver comprising at least one characteristic of the speech and/or information related to a speech disorder of said subject, and
- said initial architecture (20) further comprises a third text encoder configured to receive, as input, at least one message from a caregiver comprising at least one characteristic of the speech and/or information related to a speech disorder of said patient, and to generate, as output, a vector of text features, and
- the at least one processor is further configured to jointly train said first encoder, said second encoder, and said third encoder of said initial architecture (20), the training using a multimodality matrix having at least three dimensions defined from the vector of medical text features of said first encoder, the vector of vocal and linguistic features of said second encoder, and the vector of text features of said third encoder.

3. The device according to either one of claims **1** to **2,** wherein the training of said initial architecture (20) based on said training dataset (21) comprises, for each training sample, selecting a part of said training sample to train said initial architecture (20).

4. The device according to any one of claims **1** to **3,** wherein the demographic data comprises at least: an age, a sex, a weight, a place of residence, and/or a place of birth.

5. The device according to any one of claims **1** to **4,** wherein said prescription relates to at least one among typical or atypical antipsychotic agents prescribed in the treatment of schizophrenia, or a sedative antipsychotic, a mood stabilizer, an antiepileptic, a hypnotic, an anticholinergic, and/or benzodiazepines/anxiolytics..

6. The device according to any one of claims **1** to **5,** wherein said message (25) summarizing the clinical situation of said patient comprises at least one of the following information: demographic data, a prescription of at least one treatment, responses to at least one psychiatric questionnaire filled out by said patient, responses to at least one psychiatric questionnaire filled out by a caregiver, at least one concentration of a treatment previously measured from a blood sample of said patient or said prescription of at least one treatment.

7. The device according to any one of claims **1** to **6,** wherein said artificial intelligence system (31) for monitoring is further configured to provide, as output, a prediction of a symptomatic state of said patient in psychiatry.

8. The device according to any one of claims **1** to **7,** wherein the artificial intelligence system (31) for monitoring is further configured to provide, as output, a prediction of at least one indicator, said indicator being at least one among: a blood concentration of an antipsychotic and/or a sedative antipsychotic, a D2 occupancy rate, a presence or absence of benzodiazepines/anxiolytics, a presence or absence of anticholinergics/antidepressants, presence or absence of mood stabilizers, presence or absence of hypnotics/soporifics.

9. The device according to any one of claims **1** to **8,** wherein said pretrained foundation model is based on at least one architecture among: a ResNet-type architecture, a Hubert-type architecture, a Whisper-type architecture, a Transformer-type architecture.

10. The device according to any one of claims **1** to **9,** wherein said generated artificial intelligence system (31) is configured to receive as input at least one message summarizing the clinical situation (25) and at least one vocal signal (23) of a patient, to obtain a matching score for each pair formed between the vector of vocal and linguistic features obtained by the second encoder and each of said at least one vector of medical text features obtained by the first encoder, and to use said at least one matching score to obtain said at least one prediction of symptoms, a dosage of at least one treatment prescribed to said patient, and/or transcriptions (32).

11. A device (6) for monitoring a patient suffering from psychiatric disorders using said artificial intelligence system (31) for monitoring a patient suffering from psychiatric disorders obtained with the device (1) according to any one of claims 1 to 11, said device (6) comprising:
- at least one input configured to receive (61):
∘ at least one message (25) summarizing the clinical situation of said patient;
∘ at least one vocal signal (23) of said patient;
- at least one processor configured to:
∘ provide (62), as input to said artificial intelligence system (31), said at least one message (25) summarizing the clinical situation of said patient and said at least one vocal signal of said patient, and obtain, as output, at least one prediction of symptoms, a dosage of at least one treatment prescribed to said patient, and/or transcriptions;
- at least one output configured to provide said at least one prediction of symptoms, a dosage of at least one treatment prescribed to said patient, and/or transcriptions (32).

12. The device (6) according to claim **11,** wherein said message (25) summarizing the clinical situation of said patient is entered by a user.

13. The device (6) according to claim **11,** wherein said message (25) summarizing the clinical situation of said patient is obtained by providing clinical information (22) related to said patient as input to a pretrained biomedical language model (24), obtaining, as output, said message (25) summarizing the clinical situation of said patient, said clinical information (22) related to said patient comprising at least: a result of a psychiatric questionnaire, at least one prescription of at least one treatment, at least one concentration of a treatment previously measured from a blood sample, a prescription and/or demographic data.

14. The device (6) according to any one of claims **11** to **13,** wherein obtaining as output at least one prediction of symptoms, a dosage of at least one treatment prescribed to said patient, and/or transcriptions comprises:
• for each of the at least one message summarizing the clinical situation of said patient (25), obtaining a medical text feature vector by the first medical text encoder of said artificial intelligence system (31), said first medical text encoder receiving as input said message summarizing the clinical situation of said patient (25);
• obtaining a vocal and linguistic feature vector by the second vocal and linguistic signal encoder of said artificial intelligence system (31), said second vocal and linguistic signal encoder receiving as input said at least one vocal signal (23) of said patient;
• for each pair formed between the vocal and linguistic feature vector and each of said at least one medical text feature vector, obtaining a matching score;
• using said at least one matching score to obtain said at least one prediction of symptoms, a dosage of at least one treatment prescribed to said patient, and/or transcriptions (32).

15. A computer-implemented method for generating an artificial intelligence system (31) for monitoring a patient suffering from psychiatric disorders, said method comprising:
- receiving (41):
∘ a training dataset (21), comprising a plurality of training samples for a plurality of subjects, each training sample of said dataset (21) comprising, for each subject of said plurality of subjects:
▪ a vocal signal of said subject;
▪ clinical information related to said subject comprising at least: a result of a psychiatric questionnaire, at least one prescription of at least one treatment, at least one concentration of a treatment previously measured from a blood sample, and/or demographic data;
∘ a pretrained biomedical language model (24) (LLM) configured to receive, as input, for a patient, clinical information related to said patient comprising at least: a result of a psychiatric questionnaire, at least one prescription of at least one treatment, at least one concentration of a treatment previously measured from a blood sample, and/or demographic data; said biomedical language model (24) being further configured to generate, as output, a message (25) summarizing the clinical situation of said patient;
∘ an initial architecture (20) of said artificial intelligence system (31) for monitoring, said initial architecture (20) being configured to provide at least one prediction of symptoms, a dosage of at least one treatment prescribed to a patient in psychiatry, and/or transcriptions, said initial architecture (20) comprising:
▪ a first medical text encoder configured to receive, as input, a message (25) summarizing the clinical situation of said patient generated by said biomedical language model, and generate, as output, a vector of medical text features;
▪ a second encoder of vocal and linguistic signals configured to receive, as input, at least one vocal signal of a patient and generate, as output, a vector of vocal and linguistic features, said second encoder being obtained by a pretrained foundation model;
- generating (43) said artificial intelligence system (31) for monitoring by jointly training said first encoder and said second encoder of said initial architecture (20) based on said training dataset (21), wherein the joint training comprises iterating until convergence, for each training sample, the following steps:
∘ obtaining a message (25) summarizing the clinical situation of said patient by said biomedical language model (24) receiving, as input, at least part of the clinical information comprised in the training sample;
∘ obtaining a vector of medical text features by said first medical text encoder receiving, as input, the message (25) summarizing the clinical situation;
∘ obtaining a vector of vocal and linguistic features by said second encoder of vocal and linguistic signals receiving, as input, the at least one vocal signal comprised in the training sample;
∘ modifying the parameters of the initial architecture (20) so as to minimize a loss function based on a multimodality matrix, having at least two dimensions, defined from said vector of medical text features of said first encoder and said vector of vocal and linguistic features of said second encoder;
- providing (44) as output said artificial intelligence system (31) for monitoring.
